# EUROPEAN PATENT SPECIFICATION

(11) **EP 3 000 821 B1**
(45) Date of publication and mention of the grant of the patent: **13.03.2019**
(21) Application number: 15020001.2
(22) Date of filing: 08.01.2015
(51) Int. Cl.: C07K 1/04, C07K 14/21

(54) **Pseudofactin derivatives, a method of synthesizing pseudofactin derivatives and their use**
Pseudofactin-Derivate, Verfahren zur Herstellung von Pseudofactin-Derivaten und ihre Verwendung
Dérivés de pseudofactine, un procédé de synthèse de dérivés de pseudofactine et leur utilisation

(30) Priority: 25.09.2014 PL 40959614
(43) Date of publication of application: 30.03.2016
(73) Proprietor: Uniwersytet Wroclawski, 50-137 Wroclaw (PL)
(72) Inventor: Krasowska, Anna, 51-114 Wroclaw (PL); Lukaszewicz, Marcin, 51-351 Wroclaw (PL); Grzywacz, Daria, 80-298 Gdansk (PL); Kamysz, Wojciech, 83-210 Zblewo (PL)
(74) Representative: Kondrat, Mariusz

(56) References cited:
- EP-A1- 0 316 495
- EP-A1- 0 385 476
- US-A- 6 008 058
- US-B1- 7 211 380
- JANEK T ET AL: "Isolation and characterization of two new lipopeptide biosurfactants produced by Pseudomonas fluorescens BD5 isolated from water from the Arctic Archipelago of Svalbard", BIORESOURCE TECHNOLOGY, ELSEVIER BV, GB, vol. 101, no. 15, 1 August 2010 (2010-08-01), pages 6118-6123, XP027018557, ISSN: 0960-8524 [retrieved on 2010-03-19]
- KONNO HIROYUKI ET AL: "Synthesis and antifungal activities of cyclic octa-lipopeptide burkholdine analogues", BIOORGANIC & MEDICINAL CHEMISTRY LETTERS, PERGAMON, AMSTERDAM, NL, vol. 23, no. 14, 14 May 2013 (2013-05-14), pages 4244-4247, XP028573308, ISSN: 0960-894X, DOI: 10.1016/J.BMCL.2013.04.091
- MAÏDER PAGADOY ET AL: "Solid-Phase Synthesis of Surfactin, a Powerful Biosurfactant Produced by Bacillus subtilis, and of Four Analogues", INTERNATIONAL JOURNAL OF PEPTIDE RESEARCH AND THERAPEUTICS ; FORMERLY KNOWN AS LETTERS IN PEPTIDE SCIENCE, KLUWER ACADEMIC PUBLISHERS, DO, vol. 11, no. 3, 1 September 2005 (2005-09-01), pages 195-202, XP019287629, ISSN: 1573-3904
- JANEK TOMASZ ET AL: "Identification and characterization of biosurfactants produced by the Arctic bacteriumPseudomonas putidaBD2", COLLOIDS AND SURFACES. B, BIOINTERFACES, ELSEVIER, AMSTERDAM, NL, vol. 110, 14 May 2013 (2013-05-14), pages 379-386, XP028573153, ISSN: 0927-7765, DOI: 10.1016/J.COLSURFB.2013.05.008
- TOMASZ JANEK ET AL: "Antiadhesive activity of the biosurfactant pseudofactin II secreted by the Arctic bacterium Pseudomonas fluorescens BD5", BMC MICROBIOLOGY, BIOMED CENTRAL LTD, GB, vol. 12, no. 1, 23 February 2012 (2012-02-23), page 24, XP021117894, ISSN: 1471-2180, DOI: 10.1186/1471-2180-12-24
- JANEK TOMASZ ET AL: "Lipopeptide Biosurfactant Pseudofactin II Induced Apoptosis of Melanoma A 375 Cells by Specific Interaction with the Plasma Membrane", PLOS ONE, vol. 8, no. 3, March 2013 (2013-03), XP002736744, ISSN: 1932-6203
- MCMURRAY J S: "SOLID PHASE SYNTHESIS OF A CYCLIC PEPTIDE USING FMOC CHEMISTRY", TETRAHEDRON LETTERS, PERGAMON, GB, vol. 32, no. 36, 1 January 1992 (1992-01-01), pages 7679-7682, XP002910483, ISSN: 0040-4039, DOI: 10.1016/0040-4039(91)80563-L

## Description

This disclosure relates to pseudofactin derivatives, a novel method of their preparation by solid phase synthesis (SPPS) and their use.

*Pseudomonas fluorescens* BD5 strain, producing and secreting biosurfactant pseudofactin II, was isolated from water samples collected by the team of the Faculty of Earth Sciences and Environmental Science, University of Wroclaw, at Spitsbergen in 2005. In the laboratory of the Department of Biotransformation, Faculty of Biotechnology, University of Wroclaw, the strain was identified using the API 20E test used for the identification of *Enterobacteriaceae* and by sequencing of the 16S rRNA subunit.

A 968 bp sequence was compared with the sequences of rDNA deposited in GenBank (http://www.ncbi.nlm.nih.gov/BLAST/). The isolated strain is a Gram-negative bacteria, aerobic and rod-shaped. The optimal temperature for the cultivation of the strain on MSM substrate with 2% glucose is 28°C. The strain produces cytochrome oxidase and utilizes citrate, but does not produce β galactosidase, ornithine decarboxylase, urease, tryptophan deaminase, indole or acetoin (Janek T., Lukaszewicz M., Rezanka T., Krasowska A., (2010) Isolation and characterization of two new lipopeptide biosurfactants produced by *Pseudomonas fluorescens* BD5 isolated from water from the Arctic Archipelago of Svalbard. Bioresource Technology 101, 6118-6123).

*P. fluorescens* BD5 produces two variants of pseudofactin (known as PF I and PF II). These are cyclic lipopeptides that differ in the last amino acid at the C-terminus; valine for PF I and leucine for PF II. In this study PF II was selected for all experiments, as the amount produced was about 12 times greater than that of PF I.
The aims of the invention were to obtain pseudofactin II derivatives with special properties, developing a method for the preparation of PF II, and ensuring a high yield and purity of the products.
In particular, this disclosure relates to a pseudofactin II derivative with the following formula (A): 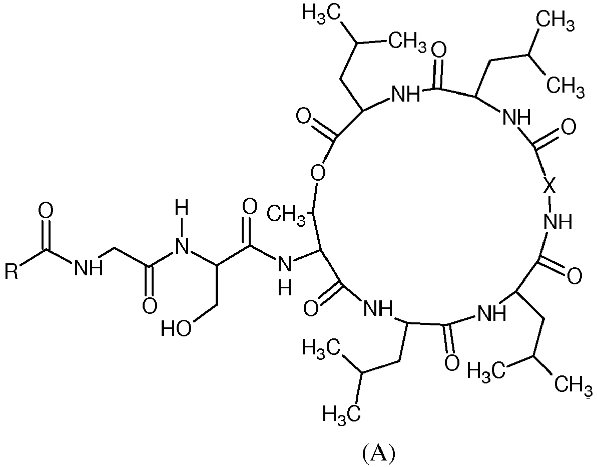 where R represents CH₃(CH₂)₁₄ to CH₃(CH₂)₂₂ and X is: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.

The subject matter of this disclosure is also the novel method of pseudofactin synthesis which comprises the following steps:
a) solid phase synthesis of the peptide R-Gly-Ser(tBu)-Thr-Leu-Leu-X-Leu-Leu-OH by the carbodiimide method, where R denotes CH₃(CH₂)₁₄ to CH₃(CH₂)₂₂ and X denotes: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val
b) cleaving the peptide from the resin while maintaining the tBu protecting group on the side chain of L-serine
c) internal cyclization of the peptide by the formation of an ester bond between the hydroxyl group of the side chain of L-threonine and the carboxyl group of the terminal L-leucine, wherein the internal cyclization is carried out in a solvent comprising of a mixture of dimethylformamide (DMF) and dichloromethane (DCM) and in the presence of a mixture of catalysts, 4-dimethylaminopyridine (DMAP), diisopropylamine (DIPEA) and N,N'- diisopropylcarbodiimide (DIC).
d) removal of the protecting groups located on the side chain of L-serine
e) purification of the peptide obtained in step d).
Advantageously, step a) according to synthesis of the pseudofactin derivatives comprises:
i. attachment of the amino acid Fmoc-L-Leu to the resin,
ii. synthesis of the linear peptide R-Gly-Ser(tBu)-Thr-Leu-Leu-X-Leu-Leu- OH,
where R denotes CH₃(CH₂)₁₄ to CH₃(CH₂)₂₂ and X denotes: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val
Advantageously, cleavage of the peptide from the resin while maintaining the protecting groups of the side chain of L-serine is performed by a solution containing 50% acetic acid and 50% toluene.

Another aim of the disclosure is the use of the pseudofactin derivative with the formula (A):
a) as carriers of hydrophobic and hydrophilic compounds into the body through contact with the skin or mucous membrane transport substances without changing their properties.
b) as compounds which reduce hydrophobicity and thus protects catheters, implants, lenses as well as tanks or reactors against colonization by microorganisms.
c) for skin washing and disinfection, washing clothes, surface cleaning, tank cleaning (e.g. with petroleum products.)
d) In the construction industry, as additives to concrete, plaster, paint and other products, in which the biosurfactant properties of the pseudofactin II derivatives may be used.
e) as a component of synergistically acting drugs, e.g. enhancing antimicrobial activity, reducing side effects such as sepsis and supporting anti-cancer action.
f) In research, as a component of chemical reagents, e.g. those used for the purification of proteins, emulsifying, transformation, stabilization and activation of enzymes.
g) As components (additives) of agricultural and food products, acting as a thickening, chelating, emulsifying, dispersing and stabilizing agent.
h) to increase the efficiency of exploitation of natural resources of , e.g. through the removal of residual oil.
i) by *in situ* bioremediation via emulsifying, chelating, and stimulation of microorganisms, leading to the biological mineralization and removal of impurities.

PF II derivatives obtained according to the invention differ in alkyl chain length or in amino acids in the cyclic part of the biosurfactant. The longer the fatty acid is in the structure of the surfactant, the lower the CMC and the higher the number of aggregations. The micellar solutions of biosurfactants are capable of solubilizing compounds, i.e. carry hydrophobic substances (hardly soluble or insoluble in water) to the biosurfactant solution at concentrations exceeding CMC. The type, composition and purity of both the solubilized substance and the biosurfactant affect the solubilization of compounds in micellar aggregates.

Natural surfactants, due to their characteristics, are involved in the pseudo-solubilization of hydrocarbons into microdroplets, facilitating the intake of hydrophobic compounds by cells. The amphiphilic structure of biosurfactants and their ability to solubilize makes them a vital factor influencing the form and durability of bacterial biofilm. They can interact with the biopolymer matrix of the biofilm, potentially leading to its dissolution by the solubilization of its components, trapping them within the micellar structure. Damage to membranes is caused by, inter alia, the incorporation of biosurfactant molecules and the resultant disruption of the transport of the various components via the membranes. There is a relationship between the length of the alkyl chain and the depth to which the compound is incorporated into the membrane. Thanks to the use of PF II analogs with different alkyl chain lengths, it is possible to create a mixture of analogs which is more effective than PF II alone.

Due to their excellent stability and solubilizing capacity, biosurfactants are used in the pharmaceutical and cosmetic industries. Emulsions containing biosurfactants may be used as carriers of hydrophobic and hydrophilic compounds. Through contact with the skin or mucous membrane, microemulsions may transport substances into the body without their properties changing.

By replacing amino acids in the cyclic part of PF II, it is possible to obtain more hydrophobic and/or more hydrophilic analogs of PF II. Materials which are used in medicine (catheters, implants, lenses), as well as in the food industry (vats, reactors), are generally hydrophobic and therefore susceptible to colonization by hydrophobic micro-organisms. By coating these materials or instruments with compounds that reduce hydrophobicity, they can be protected against colonization by microorganisms.

The synthesis of pseudofactin II derivatives involved only the L-configuration amino acids. The α-amino group of all amino acids was protected by the Fmoc group, while the side chain of L-serine was protected by the tert-butyl group (tBu). The peptide was modified by a saturated fatty acid with different chain lengths.
The derivatives may be modified in the cyclical part of the chain, although is important to use appropriate protected amino acids in the synthesis of the linear peptide.
The structure of obtained pseudofactin II derivatives is shown in the Figures below. Figure 1 shows the mass spectrum of the cyclic lipopeptide - pseudofactin with stearic acid, and Figure 2 includes a mass spectrum of the linear peptide of pseudofactin and stearic acid together with the aforementioned L-arginine (instead of L-serine) in the peptide chain. Figure 3 contains the mass spectrum of the cyclic lipopeptide - pseudofactin with stearic acid together with the aforementioned L-arginine (instead of L-serine) in the peptide chain.
The method of the invention and the use of the compounds obtained are illustrated in the following examples.

### Abbreviations:

Arg - Arginine
Gly - Glycine
DIPEA- Diisopropylethylamine
DIC - *N,N'-Diisopropylcarbodiimide*
DMAP - 4-Dimethylaminopyridine
DMF - *N,N-dimethylformamide*
DCM - Dichloromethane
Fmoc - 9-fluorenylmethoxycarbonyl
Leu - Leucine
Mir - Myristic acid
Palm - Palmitic acid
Pbf- 2,2,4,6,7-pentamethyldihydrobenzofuran-5-sulfonyl
Ser - Serine
Stea - Stearic acid
tBu- Tert-butyl
TFA - Trifluoroacetic acid
Thr - Threonine

### Example 1.

PF II derivative, modified with stearic acid, synthesized manually by solid phase peptide synthesis (SPPS) using a Fmoc/tBu strategy.

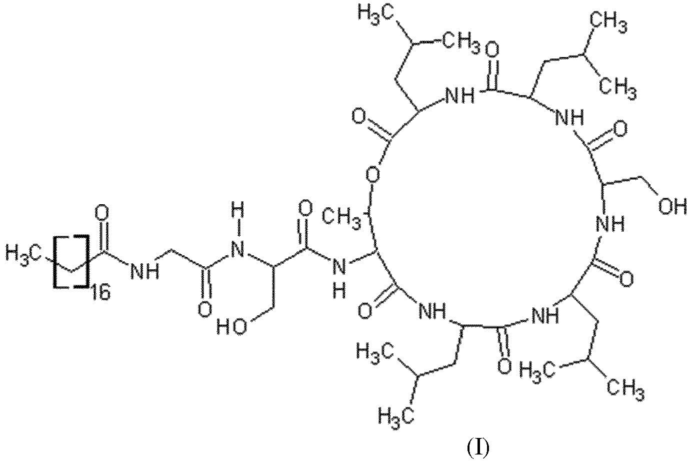

The synthesis used only the L-configuration amino acids. The α-amino group in all amino acids was protected by Fmoc, and the side chain of L-serine was protected by tert-butyl (tBu). The peptide was modified by a saturated fatty acid with eighteen carbon atoms in the molecule (stearic acid); modifications by other fatty acids, such as myristic acid, are also possible.

### Description of synthesis

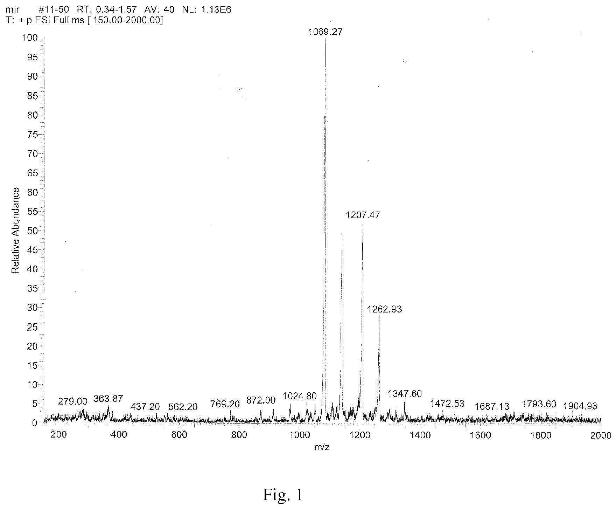

1. 0.5 g of 2-chlorotrityl resin was added to the reaction vessel. The resin was swelled in dichloromethane for 60 min. After filtration of the solvent, 0.7 mmol of Fmoc-L-Leu-OH and 1.5 mmol of DIPEA were added. The solution was stirred on a shaker overnight. After checking the loading of the amino acid (0.99 mmol/g), the solvents were filtered. Then the synthesis of the linear peptide Stea-Gly-Ser(tBu)-Thr-Leu-Leu-Ser(tBu)-Leu-Leu-OH was performed in accordance with a standard procedure which comprises:
   a) deprotection of the amino function with 20% piperidine / DMF
   b) resin wash cycle (DMF, DCM)
   c) acylation (coupling) using an equimolar mixture of the protected amino acids DIC and Oxyma Pure (Fmoc-AA : DIC: Oxyma Pure, 1:1:1). Additional amino acids derivatives were attached using a 2-fold molar excess relative to the loading of the resin, the mixture was stirred for 60 min. The progress of acylation was tested by a chloranil test.
2. In order to cleave the peptide from the resin while maintaining the side-chain protecting group for L-serine, 10 ml of a solution containing 50% acetic acid and 50% toluene was prepared.
   The peptide-resin was flooded with the solution and stirred on a magnetic stirrer for 3 h at 37 °C. Then the solution was filtered into a round bottom flask and the solvents were evaporated. A few ml of acetic acid was added to the flask and then frozen before lyophilization. The obtained compound was analyzed by TLC. Analysis conditions were as follows: DCM: methanol (5:1; v:v), the TLC plate was placed in iodine vapour.
3. For the next step (internal cyclization), 30 mg (0.026 mmol) of the obtained peptide was weighed, dissolved in 10 ml of DMF and DCM, and then 0.33 mg (0.1 eq.) DMAP, 1 µl (0.2 eq.) DIPEA and 4.5 µl of DIC (1 eq.) were added. The solution was stirred on a magnetic stirrer for 4h. The reaction and the purity of the resulting products were analyzed by TLC. After cyclization, the solvents were evaporated in a rotary vacuum. Separation of the resulting mixture of products was performed by column chromatography. The following arrangement of solvents was used as the mobile phase: DCM:MeOH (5:1; v:v).
4. The last step was to remove the protecting groups on the side chain of the obtained peptide. The lyophilized compound was dissolved in a mixture of TFA:H₂O (19:1, 700 µl) and stirred on a magnetic stirrer for 60 min. After that time, the solvents were evaporated, water was added and the mixture was lyophilized. The resulting compound was purified by column chromatography. The column was filled with silica gel (Kiesgel MN-60) with a particle size <0.08 mm. 10 g of silica gel was used per 0.1 g of the reaction product mixture. The following system of solvents was used as the mobile phase: DCM: MeOH: AcOH (5:1:0.5; v:v:v). The obtained oily product was purified, obtaining 15 mg of pure peptide (75% yield). The procedure was confirmed by mass analysis (signals m/z: 1069.27 [M]). The results are shown in FIG. 1.

### Example 2.

The synthesis of analogs with different D- and L-configurations of the used amino acid is carried out in a manner analogous to that described above, except that in the synthesis of the protected linear peptide, a protected amino acid with a suitable configuration should be used.

### Example 3.

Synthesis of derivatives with a different amino acid composition of the lipopeptide are carried out in a manner analogous to that described above, except that in the synthesis of the linear peptide, an appropriate protected amino acid should be used.

Lipopeptide PF II was synthesized manually by solid phase peptide synthesis (SPPS) using a Fmoc/tBu strategy. The synthesis involved only the L-configuration amino acids. The α-amino group of all amino acids was protected by the Fmoc, the side chain of L-serine was protected by the tert-butyl group (tBu), whereas the side chain of L-arginine was protected by the Pbf group. The peptide was modified by a saturated fatty acid with eighteen carbon atoms (stearic acid).

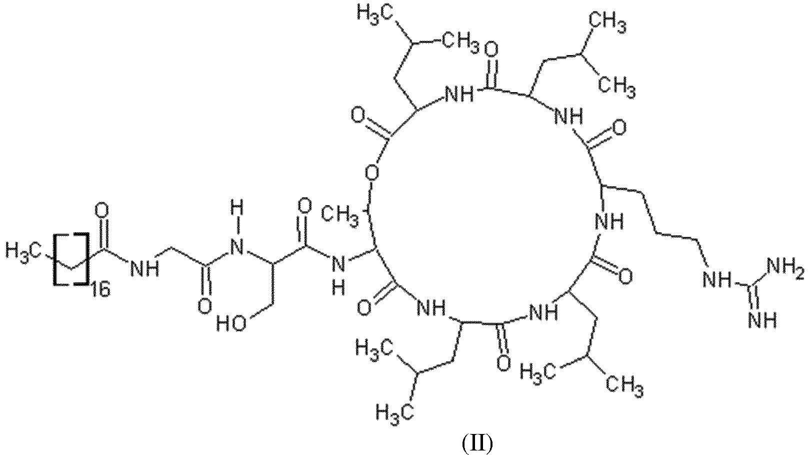

### Peptide production:

a) solid phase synthesis of the peptide Stea-Gly-Ser(tBu)-Thr-Leu-Leu-Arg(Pbf)-Leu-Leu-OH using the carbodiimide method on 2-chlorotrityl resin
b) cleavage of the peptide from the resin (while maintaining the tBu protecting group on the side chain of L-serine and the Pbf protecting group on the side chain of L-arginine)
c) internal cyclization of the peptide (formation of an ester bond between the hydroxyl group on the side chain of L-threonine and the carboxyl group of L-leucine)
d) removing the protecting groups located on the side chain of L-serine and L-arginine
e) purification of the obtained peptide.

### Description of synthesis

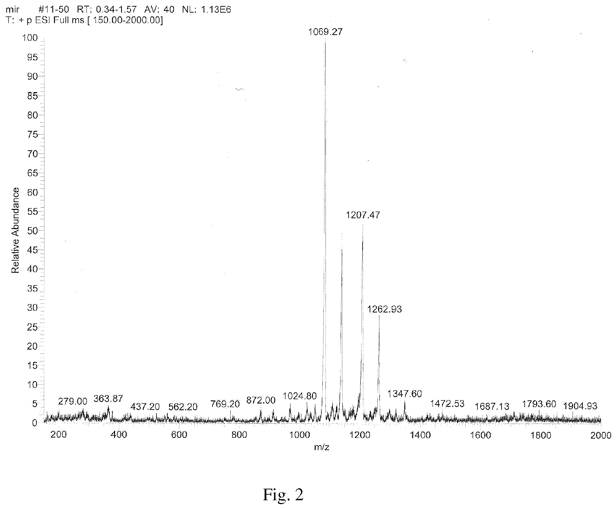

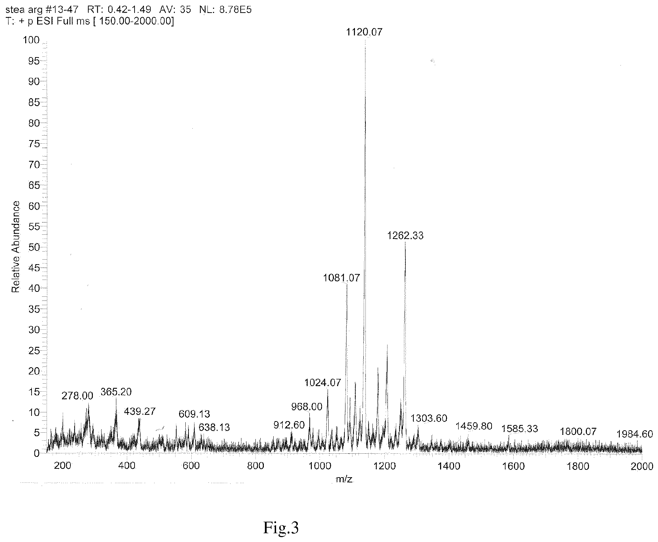

1. 0.5 g of 2-chlorotrityl resin was added to the reaction vessel. The resin was swelled in dichloromethane for 60 min. After filtration of the solvent, 0.7 mmol of Fmoc-L-Leu and 1.5 mmol of DIPEA were added. The solution was stirred on a shaker overnight. After checking the loading of the amino acid (0.99 mmol/g), the solvents were filtered. Then the synthesis of the linear peptide Stea-Gly-Ser(tBu)-Thr-Leu-Leu-Arg(Pbf)-Leu-Leu-OH was performed in accordance with a standard procedure which comprises:
   a) deprotection of the amino function with 20% piperidine / DMF
   b) resin wash cycle (DMF, DCM)
   c) acylation (coupling) using an equimolar mixture of the protected amino acids DIC and Oxyma Pure (Fmoc-AA : DIC: Oxyma Pure, 1:1:1). Additional amino acid derivatives were attached using a 2-fold molar excess relative to the loading of the resin, the mixture was stirred for 60 min. The progress of acylation was tested by a chloranil test.
2. In order to cleave the peptide from the resin while maintaining the side-chain protecting group for L-serine, 10 ml of a solution containing 50% acetic acid and 50% toluene was prepared. MALDI-TOF mass analysis was also performed (signals found: m/z 1447.53 [M]).
   The results are presented in FIG. 2.
   The peptide-resin was flooded with the solution and stirred on a magnetic stirrer for 3 h at 37°C. Then the solution was filtered into a round bottom flask and the solvents were evaporated. A few ml of acetic acid was added to the flask and then frozen before lyophilization. The obtained compound was analyzed by TLC. Analysis conditions were as follows: DCM: methanol (5:1; v:v), the TLC plate was placed in iodine vapor.
3. For the next step (internal cyclization), 30 mg (0.026 mmol) of the obtained peptide was weighed, dissolved in 10 ml of DMF and DCM, and then 0.33 mg (0.1 eq.) DMAP, 1 µl (0.2 eq.) DIPEA and 4.5 µl of DIC (1 eq.) were added. The solution was stirred on a magnetic stirrer for 4h. The reaction and the purity of the resulting products were analyzed by TLC. After cyclization, the solvents were evaporated in a rotary vacuum. Separation of the resulting mixture of products was performed by column chromatography. The following arrangement of solvents was used as the mobile phase: DCM:MeOH (5:1; v:v).
4. The last step was to remove the protecting groups on the side chain of the obtained peptide. The lyophilized compound was dissolved in a mixture of TFA:H₂O (19:1, 700 µl, and stirred on a magnetic stirrer for 60 min. After that time, the solvents were evaporated, water was added and the mixture was lyophilized. The resulting compound was purified by column chromatography. The column was filled with silica gel (Kiesgel MN-60) with a particle size <0.08 mm 10 g of silica gel was used per 0.1 g of the reaction product mixture. The following system of solvents was used as the mobile phase: DCM: MeOH: AcOH (5:1:0.5; v:v:v). The obtained oily product was purified, obtaining 15 mg of pure peptide (75% yield). The procedure was confirmed by mass analysis (signals m/z: 1120.03 [M]). The results are shown in FIG. 3.

### Example 4.

Microorganisms were cultured on either liquid or solid media prepared in deionized water. The experiment was performed using the following bacteria: *Escherichia coli, Enterococcus faecalis, Enterococcus hirae, Staphylococcus aureus* and *Staphylococcus epidermidis,* cultured for 24 h in LB medium at 37°C, and the yeast *Candida albicans,* grown in YNB medium at 28°C. The strains were taken from a frozen state (-80°C) and inoculated on LB medium (bacteria) and YPG medium (yeast). After 24 or 48 hours of incubation at 28°C or 37°C the cultures were prepared for further experiments. 5 ml of liquid LB or YNB medium was inoculated with bacteria or yeast and incubated at 28°C or 37°C for 22-24 hours.

Bacterial and yeast biofilms were formed on plastic Thermanox discs with a diameter of 13 mm, on glass coverslips 10 mm in diameter, and on cut segments of silicone catheters, arranged in a 24-well plate. The prepared wells were loaded with 500 µl of bacterial suspension in LB and yeast in RPMI-1640 with an optical density OD₆₀₀=0.01 and 0.25 mg/ml of pseudofactin II. Control wells contained no pseudofactin II. After 24 hours of incubation at 37°C, biofilms were gently rinsed in PBS and incubated for 30 minutes at 37°C in 1 ml of PBS containing 0.6% of Live/Dead BacLight viability kit (Molecular Probes, Eugene, OR) for bacteria, and Concanavalin A-Alexa Fluor 488 conjugate (Molecular Probes) at 25 µg/ml for yeast. After that time the solutions of the dyes were removed, and the biofilm was rinsed twice with PBS. The preparations on the slides were then observed under a scanning confocal microscope Olympus Fluoview 500 (Olympus Optical Co.Ltd., Japan). Preparations were analyzed and photographed.

In experiments on microbial colonization of catheters in the presence of pseudofactin II, 10 µl inoculum of the tested strains was transferred to 1000 µl of fresh media, LB and YNB. Then 1000 µl of pseudofactin II solution was added to the suspensions. In order to prepare biofilm on catheters we placed 4 cm long segments of catheters in the cell suspension and incubated for 24 h at 37°C. After 24 h, the portions of catheters were flushed with distilled water. After rinsing, the catheters were stained with 0.1% crystal violet solution for 20 minutes and then washed three times with distilled water, then allowed to dry at room temperature.

In a parallel experiment, fragments of catheters were placed in sterile tubes with 2 ml of pseudofactin II solution in PBS at a concentration of 0.25 mg/ml and incubated for 2 h at 37°C. After 2 hours, the catheters were rinsed twice with PBS. Then, after the addition of 2 ml of inoculum the catheters were incubated at 37°C for 24 hours. The experiment was carried out twice, and staining with 0.1% crystal violet was performed as in the previous paragraph.

The obtained results show an inhibitory effect of pseudofactin II analogs on the formation of bacterial and yeast biofilm under aerobic conditions. The pseudofactin II analogs added to the samples resulted in a very strong reduction (from 90 to 95%) of biofilm formation on plastic, glass and silicone surfaces. 100% of microorganisms occurred in the samples without the use of biosurfactants, and in samples containing PF II analogs (final concentration 0.25 mg/ml) 0% to 5% microorganisms were observed, depending on the type of the analog and the tested microbial strain.

## Claims

1. A pseudofactin II derivative with the following formula (A): 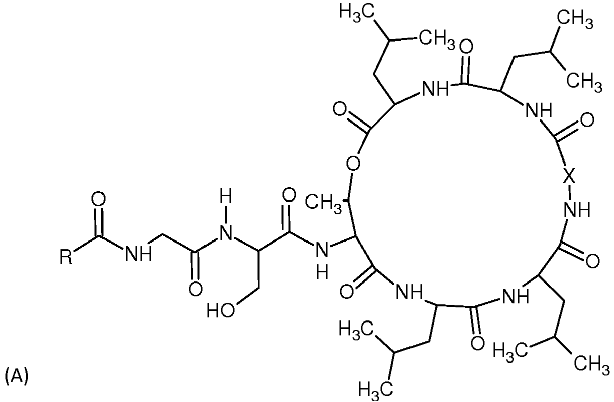 where R denotes 1CH₃(CH₂)₁₆ and X is: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Thr(tBu), Trp(Boc), Tyr(tBu), Val.

2. A method of pseudofactin synthesis comprising the following steps:
a) solid phase synthesis of the peptide R-Gly-Ser(tBu)-Thr-Leu-Leu-X-Leu-Leu-OH by the carbodiimide method, where R denotes CH₃(CH₂)₁₆ and X denotes: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Thr(tBu), Trp(Boc), Tyr(tBu), Val.
b) cleaving the peptide from the resin while maintaining the tBu protecting group on the side chain of L-serine
c) internal cyclization of the peptide by the formation of an ester bond between the hydroxyl group of the side chain of L-threonine and the carboxyl group of the terminal L-leucine, wherein the internal cyclization is carried out in a solvent consisting of a mixture of dimethylformamide (DMF) and dichloromethane (DCM) and in the presence of the mixture of catalysts 4-dimethylaminopyridine (DMAP), diisopropylamine (DIPEA) and N,N'-diisopropylcarbodiimide (DIC).
d) removal of the protecting groups located on the side chain of L-serine
e) purification of the peptide obtained in step d).

3. The method of synthesis of the pseudofactin II derivatives, according to claim 2, **characterised in that** the step a) comprises:
i. attachment of the amino acid Fmoc-L-Leu to the resin,
ii. synthesis of the linear peptide R-Gly-Ser(tBu)-Thr-Leu-Leu-X-Leu-Leu- OH, where R denotes CH₃(CH₂)₁₆ and X denotes: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Thr(tBu), Trp(Boc), Tyr(tBu), Val.

## Patentansprüche

1. Pseudofactin-Derivat II der folgenden Formel (A): 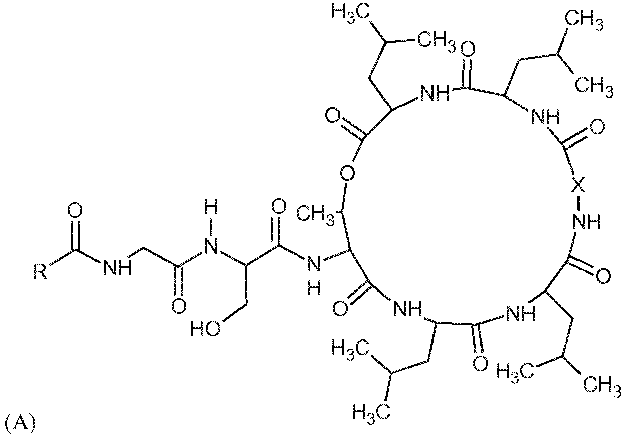 worin R ist CH₃(CH₂)₁₆
und X bedeutet: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.

2. Verfahren zur Synthese der Pseudofactin, das folgende Stufen umfasst:
a) Festphasensynthese des Peptids R-Gly-Ser(tBu)-Thr-Leu-Leu-X-Leu-Leu-OH nach der Carbodiimid-Methode, worin R ist CH₃(CH₂)₁₆ und X bedeutet: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.
b) Abspaltung des Peptids von dem Harz, wobei gleichzeitig die Schutzgruppe tBu an der L-Serin-Seitenkette verbleibt
c) Interne Zyklisierung des Peptids durch Bildung einer Esterbindung zwischen Hydroxylgruppe der L-Threonin-Seitenkette und der Carboxylgruppe vom terminalen L-Leucin, in der die interne Zyklisierung in einem Lösungsmittel, das eine Mischung aus Dimethylformamid und Dichlormethan umfasst, und bei dem Vorhandensein von folgenden Katalysatoren erfolgt: 4-Dimethylaminopyridin (DMAP), Diisopropylethylamin (DIPEA) und N,N'-Diisopropylcarbodiimid (DIC).
d) Entfernung der Schutzgruppen an der L-Serin-Seitenkette
e) Reinigung des Peptids, das in Stufe d) hergestellt wird.

3. Verfahren zur Synthese der Pseudofactin-Derivate II nach Anspruch 2, **dadurch gekennzeichnet, dass** die Stufe a) Folgendes umfasst:
i. Koppeln der Aminosäure Fmoc-L-Leu mit Harz,
ii. Synthese des linearen Peptids R-Gly-Ser(tBu)-Thr-Leu-Leu-X-Leu-Leu-OH, worin R ist CH₃(CH₂)₁₆
und X bedeutet: Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.

## Revendications

1. Dérivée de pseudofactine II de formule suivante (A) : 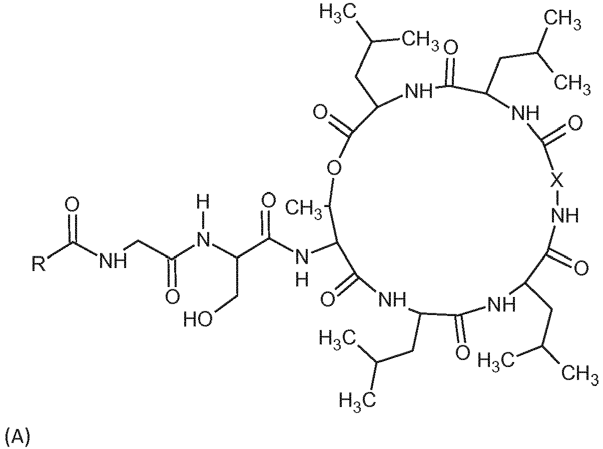 où R signifie CH₃(CH₂)₁₆
et X signifie : Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.

2. Procédé de synthèse de pseudofactine comportant les étapes suivantes :
a) synthèse en phase solide du peptide R-Gly-Ser(tBu)-Thr-Leu-Leu-X-Leu-Leu-OH par la méthode carbodiimide, où R signifie CH₃(CH₂)₁₆ X signifie : Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.
b) clivage du peptide de la résine,tout en maintenant le groupe protecteur tBu sur la chaîne latérale de L-sérine
c) cyclisation interne du peptide par formation de la liaison ester entre la fonction hydroxyle sur la chaîne latérale de L-tréonine et la fonction carboxylique de L-léucine, dont la cyclisation interne est conduite dans le solvent contenant le mélange du diméthylformamide (DMF) et du dichlorométhane et en présence du mélange catalytique : 4-diméthylaminopyridine (DMAP), diisopropylamine (DIPEA) et N,N'-diisopropylcarbodiimide (DIC).
d) suppression des groupes protecteurs disposés sur la chaîne latérale de L-sérine
e) lavage du peptide obtenu à l'étape d).

3. Procédé de synthèse des dérivés de pseudofactine II selon la revendication 2, **caractérisé en ce que** l'étape a) comprend :
i. addition de l'acide Fmoc-L-Leu à la résine,
ii. synthèse du peptide linéaire R-Gly-Ser(tBu)-Thr-Leu-Leu-X-Leu-Leu-OH, où R signifie CH₃(CH₂)₁₆ et X signifie : Ala, Arg(Pbf), Asn(Trt), Asp(OtBu), Cys(Trt), Glu(OtBu), Gln(Trt), Gly, His(Trt), Ile, Leu, Lys(Boc), Met, Phe, Pro, Ser(tBu), Thr(tBu), Trp(Boc), Tyr(tBu), Val.
